(19) Europäisches Patentamt
European Patent Office
Office européen des brevets

(11) Publication number: **0 437 092 A1**

(12) **EUROPEAN PATENT APPLICATION**

(21) Application number: 90314249.5

(22) Date of filing: 24.12.90

(51) Int. Cl.⁵: **C12Q 1/68**, G01N 33/532, G01N 33/58

(30) Priority: 25.12.89 JP 332611/89

(43) Date of publication of application:
17.07.91 Bulletin 91/29

(84) Designated Contracting States:
DE FR GB IT

(71) Applicant: **Kabushiki Kaisha Toshiba
72, Horikawa-cho Saiwai-ku
Kawasaki-shi (JP)**

(72) Inventor: **Ishimori, Yoshio
1-1 Shibaura, 1-chome, Minato-ku
Tokyo 105 (JP)**

(74) Representative: **Freed, Arthur Woolf et al
MARKS & CLERK 57-60 Lincoln's Inn Fields
London WC2A 3LS (GB)**

(54) **Gene diagnosis.**

(57)    Gene diagnosis to determine the objective DNA is disclosed, which is performed by preparing single-stranded gene (3) from a sample, making the single-stranded gene (3) to react with RNA gene probe (4) which specifically reacts with the objective DNA in order to form DNA-RNA hybrid (5), making the DNA-RNA hybrid (5) to react with liposome agent (6) for gene detection, where the liposome agent (6) has a part of monoclonal antibody or RNase H immobilized on the surface of the liposome which is formed of a phospholipid and which contains a fluorescent material as a marker material therein, making a composite of the DNA-RNA hybrid and the liposome agent to react with anti-nucleic acid antibody (7) and complement (8), and breaking the liposome agent (6) to detect the marker material flowed out.

EP 0 437 092 A1

F I G. 1

## GENE DIAGNOSIS

This invention relates to gene diagnosis which is performed by detecting a specific gene existing in a sample.

The gene information stored in DNA is expressed in the form of proteins or enzymes by means of messenger RNA. Functions of the proteins and the enzymes are to produce various kinds of compounds, which form an organism. The total number of human genes is said to be 50,000 to 100,000. If any abnormalities or variations, such as deletion and duplication, are caused in the genes, proteins are produced which are made to change in characteristic, kind, and amount. These changes agitate somatic balance of an organism, and thus cause genetic diseases. Therefore, by detecting an etiological gene, the diseases can be inversely identified or prevented. In the same manner, a disease due to a pathogen not a gene can be also identified or prevented by detecting a gene of the pathogen.

In the recent years, a diagnosis based on gene itself (which is called gene diagnosis) has become possible, as biochemistry advances. Some specific features are noticed in the gene diagnosis, as compared with conventional diagnoses.

Considering a mechanism of appearance of a disease, either a change on genes or infection of a pathogen precedes a biological change. Therefore, gene diagnosis can advantageously diagnose or predict a disease preceding an onset, i.e. in latency phase or in a very early stage of the disease.

Additionally, a genetic disease can be diagnosed independently of an organ or tissues analyzed, because all genes in a living body are identical. This is especially advantageous for fetus diagnosis, because it can be performed by taking amniotic fluid from a pregnant woman and examining fetal cells suspended in the fluid.

General gene diagnosis is briefly described as follows ; extracting genes from a sample, cleaving the genes with suitable restriction enzymes into fragments as needed, performing electrophoresis and Southern-blotting, making a gene probe labeled with radioisotopes which correspond to the objective gene to hybridize, and making the hybrid to sensitize on a X-ray film at low temperature to confirm existence of the objective gene. However, since this method requires radioisotopes, a place for diagnosis is restricted and much care should be taken for dealing with chemical agents.

To eliminate these defects, a safe label agent is expected to develop which substitutes radioisotopes. Such agents as using avidin-biotin linkage, enzymes, or fluorescent materials have been already proposed. However, these agents cannot be compared with radio-isotope in sensitivity. Moreover, they require at least several days for the gene detection, and their measurements are rather complicated.

An object of the invention is to propose gene diagnosis which can be performed by safe and easy steps in a short time, and can detect the objective gene at high sensitivity.

Gene diagnosis of the present invention is composed of a step of preparing a single-stranded gene from a sample, a step of making RNA or DNA probe which specifically reacts with the objective DNA or RNA to react to said single-stranded gene so as to form DNA-RNA hybrid, a step of making the obtained DNA-RNA hybrid to react with liposome agent for gene detection, where said liposome agent comprises a liposome which is formed of phospholipid and/or glycolipid and contains a marker material therein, and a substance immobilized on the surface of the liposome which specifically recognizes DNA-RNA hybrid, DNA, or RNA, and a step of making the composite of said DNA-RNA hybrid and the liposome agent to react with anti-nucleic acid antibody and complement in order to detect the marker which is flowed out when said liposome is broken.

This invention can be more fully understood from the following detailed description when taken in conjunction with the accompanying drawings, in which :

Fig. 1 shows gene diagnosis according to the present invention ;

Fig. 2 shows an automatic apparatus for gene detection of the present invention ; and

Fig. 3 is a graph showing relative fluorescence intensity obtained by a reaction between the liposome agent according to the present invention and rabbit anti-mouse IgG antibody solution.

In the present invention, a step of preparing a single-stranded gene from a sample is performed according to a general method (See, for example, "Rinsho Kenso (or Clinical Test)", Vol. 32, No. 4, pp. 401 (1988)). In this method, a double-stranded gene is extracted from a sample solution and cleaved with a restriction enzyme for fragmentation. Then a single-stranded gene is prepared therefrom by e.g. denaturation.

An RNA or DNA gene probe used in the invention comprises a base sequence which is complementary to a specific base sequence of the objective DNA or RNA. If the objective DNA or RNA exists in the single-stranded gene prepared as above-mentioned, the RNA or DNA gene probe specifically reacts with the objective DNA or RNA to form DNA-RNA hybrid. This probe requires no radioisotope as a labeling agent.

A liposome agent according to the invention is described in detail as follows. Inventors of the present invention have already disclosed in Japanese Unexamined Patent Disclosure No. 60-117159 a liposome agent for immune analysis comprises a liposome which is formed of a lipid membrane and contains a hydrophilic marker material (e.g. fluorescent material) therein and which has antibodies or antigens immobilized on the surface of the liposome. Immune analysis utilizing this agent is performed as follows. The liposome agent is added into a sample containing antigens or antibodies, and complements are further added thereto. As a result, the liposome is made to break because of the antigen-antibody reaction with the function of complement, which causes the marker material to flow out of the liposome. Since correlation is confirmed between the amount of the material flowed out and that of the substance to be detected in the sample, the substance can be detected by determining the marker material according to a given method (e.g. fluorescent analysis). By utilizing this agent, the signal amplifying effect is obtained, which accompanies a large amount of marker material flowed out. Therefore, high sensitivity is given, and easy analysis steps can be expected. When this liposome agent can be utilized as an agent for gene detection, high sensitivity is obtained which is compared to that obtained when utilizing radioisotope as a labeling agent. Moreover, a safe and easy manipulation is possible.

In the liposome agent for gene detection of the invention, the liposome is mainly formed of a phospholipid and/or a glycolipid. Examples of phospholipids and glycolipids are dipalmitoylphosphatidylcholine (DPPC), distearoylphosphatidylcholine (DSPC), dipalmitoylphosphatidylethanolamine (DPPE), dioleoylphosphatidylethanolamine (DOPE), dimyristoylphosphatidylethanolamine (DMPE), and distearoylphosphatidylethanolamine (DSPE), but are not restricted thereto. A fatty acid carbon chain in these phospholipids and glycolipids preferably has 12-18 carbons, and even number of carbons are more preferable. Cholesterol can be added in 10-500 molar percent to the phospholipids and the glycolipids as needed, which leads to prepare a stable multilamellar liposome.

In the liposome agent for gene detection of the invention, a marker material contained in the liposome is selected from materials which is hydrophilic and can be determined when flowing out of the liposome. Examples of these materials are fluorescent materials such as carboxyfluorecsein which shows no fluorescence by quenching in high concentration, but shows intense fluorescence in low concentration ($10^{-3}$ M or less) ; luminescent materials such as luminol and luciferin which shows luminescent by oxidation out of the liposome ; light-absorbable materials such as water-soluble pigments having specific absorption bands within visible range or ultraviolet range ; glycosides such as glucose and sucrose which is decomposed by oxidation enzymes to cause oxygen consumption or produce peroxides, and their oxidation enzymes ; ionic compounds such as tetrapentylammonium which is relatively high molecular weight ; coenzymes such as nicotinamide adenine dinucleotide (NAD) ; and radical compounds such as methylviologen. The marker material is selected from these compounds depending on a method for detecting the material, sensitivity, stability of the liposome, and so on.

In the liposome agent for gene detection utilized in the invention, a material which is immobilized on the surface of the liposome and which specifically recognizes DNA-RNA hybrid, DNA, or RNA is exemplified an antibody or a part of an antibody, or an enzyme or a part of an enzyme. The antibody may be of any class of IgG, IgE, IgD, IgA, and IgM, and subclasses. To improve sensitivity, monoclonal antibodies are preferable rather than polyclonal ones. Also, F(ab')$_2$ obtained by removing the Fc fragment from the antibody may be used, and Fab' obtained by reducing the Fab' with a reductant may also be used. For example, an monoclonal antibody prepared by utilizing a mouse according to the method of W.D. Stuart (Proc. Natl. Acad. Sci. USA.", Vol. 78, No. 6, pp. 3751-3754, June, 1981) may be utilized. The enzyme may be RNase or DNase, for example, RNase H or S$_1$-nuclease. RNase is more preferable because it specifically recognizes DNA-RNA hybrid. Further, an enzyme which is deactivated the activity as a nuclease is more preferable.

A method for producing a liposome agent is described as follows. In this example, halogenated acetyl group is utilized as a functional group for immobilizing an antibody, an enzyme or a part of them on the surface of the liposome.

First, halogenated acetyl group represented by the following formula is introduced to the desired phospholipid or glycolipid ;

$$-CO(CH_2)_mNHCOCH_2X$$

(wherein m represents an integer of 0-12, and by changing m, a length of a spacer i.e. a carbon chain which combines a lipid molecule and the functional group can be selected. X is $C\ell$, Br, or I).

For example, amino group of ω-amino acid such as 3-aminopropionic acid ($NH_2(CH_2)_2COOH$) or 5-aminovaleric acid ($NH_2(CH_2)_4COOH$) is protected with t-butoxycarbonyl group (hereinafter abbreviated Boc group), etc. After that, the product, N-hydroxysuccinimide (HSI), and N,N'-dicyclohexylcarbodiimide (DCCD) are reacted with a lipid containing amino group (e.g. DPPE) in the presence of triethylamine (TEA), followed by removing the Boc group with hydrochloric acid, etc. Also, amino group of ω-amino acid may be protected with Boc group, reacted with HSI and DCCD in the presence of TEA to produce succinimide ester, which is reacted with a lipid containing amino group, followed by removing the Boc group. Next, the obtained lipid with the spacer is reacted with halogenated acetic acid, HSI, and DCCD in the presence of TEA.

Also, carboxyl group of ω-amino acid may be protected by esterification in advance, combined with a halogenated acetic acid, followed by removing the protecting group, and reacted with a lipid containing amino group, HSI, and DCCD in the presence of TEA.

Resultant lipid can be easily purified utilizing a thin-layer chromatography for separation.

Next, an adequate amount of phospholipid and/or glycolipid containing —CO(CH₂)ₘNHCOCH₂X group, cholesterol, and other lipids as needed are placed into a flask. A solvent is further added into the flask for dissolution and mixing. Then the solvent is removed to form a lipid thin layer onto the inner surface of the flask. A solution containing a proper marker material is further added, and then heated to an adequate temperature, sealed, and stirred violently to prepare a suspension of multilamellar liposome.

On the other hand, SH group is introduced to a substance which specifically recognizes DNA-RNA hybrid, DNA, or RNA, by utilizing an enzyme such as pepsin or by means of reduction. The substance may also be reacted with a bifunctional agent such as N-succinimidyl-3-(2-pyridyl) dithiopropionate (SPDP) and reduced to introduce the SH group.

The liposome suspension is made to react in a proper buffer with the substance which specifically recognizes DNA-RNA hybrid, DNA, or RNA, thereby immobilizing the substance on the surface of the liposome via —CO(CH₂)ₘNHCOCH₂S linkage.

In the present invention, anti-nucleic acid antibody, e.g. rabbit anti-nucleic acid antibody can be prepared by a general method (See, for example, "Protein, Nucleic Acid, and Enzyme", temporary special edition, Vol. 11, No. 15, pp. 1452-1457 (1966)). Complement, e.g. serum of guinea pig is adequately diluted for use.

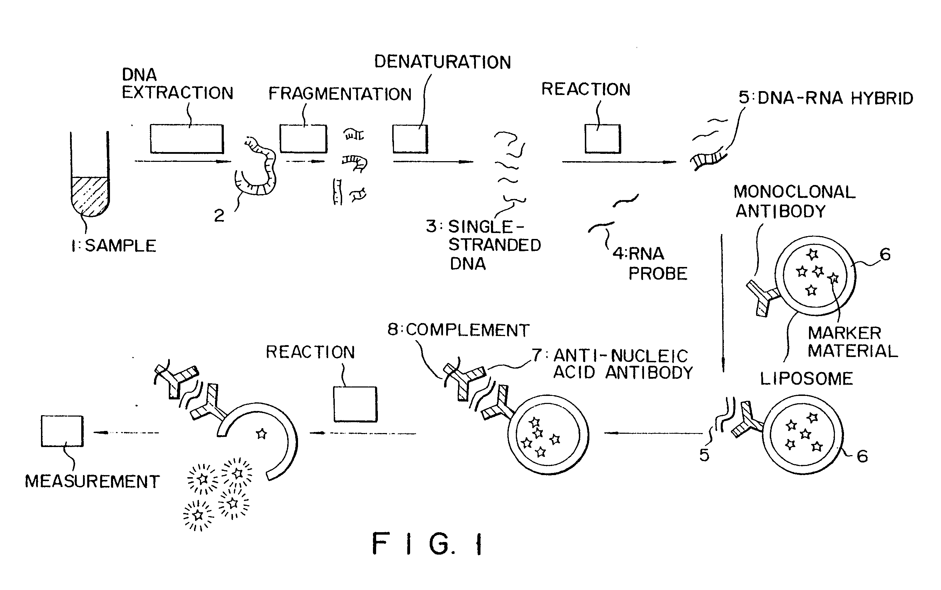

One example of gene diagnosis of the present invention is described in detail according to Fig. 1 as follows. In this example, hepatitis B virus (HBV) gene in human serum is detected.

First, double-stranded DNA 2 is extracted from a sample solution (serum) 1, cleaved with a proper restriction enzyme for fragmentation, and denatured to prepare single-stranded DNA 3. This operation is preferably performed at least 70°C in order to avoid self-hybridization among the single-stranded DNA 3. The obtained solution is neutralized, cooled to 50-60°C, and reacted with RNA gene probe 4 against the HBV gene in the presence of 50% formaldehyde. If the sample solution 1 contains HBV gene to be detected, DNA-RNA hybrid 5 is formed. Next, the resultant solution is diluted with a proper buffer, added with a suspension of the liposome agent 6, and reacted for a predetermined time. Rabbit anti-nucleic acid antibody 7 and complement 8 having a proper concentration are further added to the suspension, and the obtained mixture is reacted for a predetermined time. If the mixture contains the DNA-RNA hybrid 5, the liposome agent 6 is broken, and marker material therein is flowed out. Therefore, by determining the marker material flowed out utilizing an adequate method (e.g. fluorescence spectrophotometry, etc.), the existence of the HBV gene can be confirmed.

Here, conditions such as time, temperature, and pH for the complete reaction between the liposome agent and the test sample gene vary, depending on characteristics of the test gene and the corresponding gene probe (e.g. length, base sequence, etc.), characteristics of the liposome agent, and characteristics, amount, and purity of a substance combined to phospholipid or glycolipid, which specifically recognizes the DNA-RNA hybrid, DNA, or RNA. Optimum conditions and buffer solutions are thus preferably determined in each case.

According to gene diagnosis of the invention, high sensitivity can be obtained which is compared to that obtained in a conventional method utilizing radioisotope as a labeling agent of gene probe, due to an amplifying effect caused by a large amount of marker material which is flowed out of the liposome. Since no radioisotope is required, a safe manipulation can be obtained with no restricted places. Moreover, it is not required to separate DNA-RNA hybrid, which leads to a short period of time for determination and make it easy to manufacture an automatic apparatus. Further, since this liposome can be used independently of the objective genes, costs for agent development can be greatly reduced.

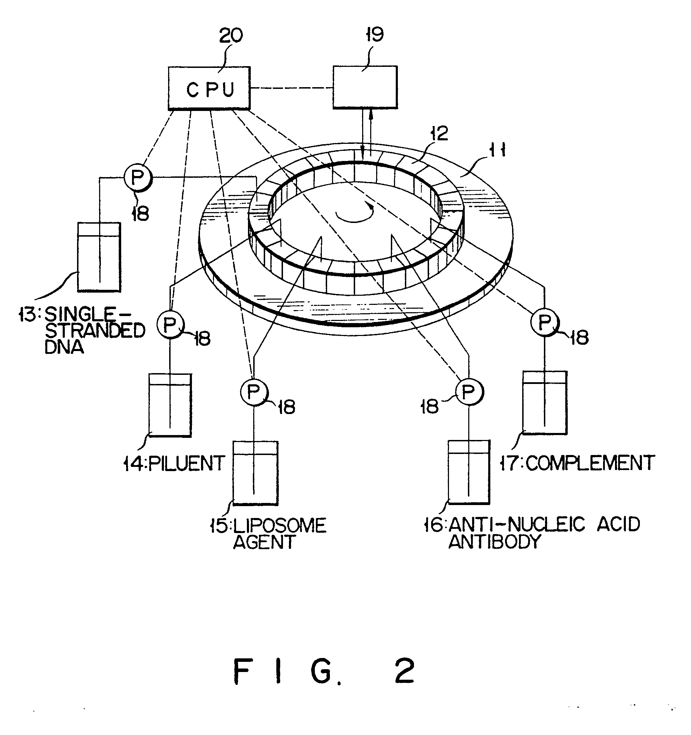

Fig. 2 shows an automatic apparatus for gene detection in order to conduct the method of the present invention. A plurality of reaction cell 12 are arranged annularly on turntable 11. Turntable 11 is rotated intermittently. RNA gene probe solution is placed in each reaction cell 12. Single-stranded DNA solution 13, diluent 14, suspension of liposome agent 15, anti-nucleic acid antibody solution 16, and complement solution 17 are sequentially supplied via pump 18 to each reaction cell 12 following the rotation of turntable 11. The solution after the reaction is measured with fluorescence spectrophotometer 19 at a predetermined position. The movement of pump 18 and fluorescence spectrophotometer 19 is program-controlled with CPU 20.

Description of examples according to the invention is as follows.

## Example 1

Dipalmitoylphosphatidylcholine (DPPC), dipalmitoylphosphatidylethanolamine (DPPE), and cholesterol utilized in this example are manufactured by Sigma Co. Other agents used in this example are commercially available extra fine grade and not purified. All water used is ion exchange water.

(A) Preparation of liposome agent having immobilized monoclonal anti-DNA-RNA hybrid recognizing antibody

[1] Synthesis of $NH_2$-$(CH_2)_4CO$-DPPE(or $NH_2$-$C_5$-DPPE)

(a) Synthesis of Boc-5-aminovaleric acid

3 $m\ell$ (ca. 20 mmol) of triethylamine (TEA) and 10 $m\ell$ of water were added to 1.17 g (10 mmol) of 5-aminovaleric acid (manufactured by Aldrich Co.) for dissolution. The resultant solution was added with a solution which was prepared by dissolving 2.7 g (11 mmol) of 2-(t-butoxycarbonyloxyimino)-2-phenylacetonitrile (manufactured by Peptide Laboratory, trade name : Boc-ON) in 10 $m\ell$ of dioxan, and the obtained mixture was stirred at room temperature for three hours, thereby protecting an amino group of 5-aminovaleric acid with a Boc group. After the reaction, the obtained solution was concentrated with a rotary evaporator, and sequentially extracted with ethyl acetate, 5% sodium hydrogencarbonate solution, and 5% citric acid solution for purification. Lastly, the resultant solution was dehydrated with anhydrous sodium sulfate, and crystallized at a low temperature to obtain boc-5-aminovaleric acid. Yield was ca. 70%.

(b) Synthesis of boc-5-aminovaleric acid succinimide ester

0.23 g (1 mmol) of Boc-5-aminovaleric acid was suspended in 20 $m\ell$ of chloroform, and the obtained suspension was further added with 0.13 g (1.1 mmol) of N-hydroxysuccinimide (HSI ; manufactured by Peptide Laboratory) and 0.25 (1.2 mmol) of dicyclohexylcarbodiimide (DCCD ; manufactured by Peptide Laboratory). The obtained mixture was stirred at room temperature for three hours. After reaction, a solvent in the mixture was removed with a rotary evaporator, and the resultant product was added with 30 $m\ell$ of ethyl acetate for dissolution. This solution was filtered to remove a precipitant. A solvent in the resultant solution was removed again, and the product was dissolved in 5 $m\ell$ of chloroform in order to obtain Boc-5-aminovaleric acid succinimide ester solution (supposed ca. 0.2 mmol/$m\ell$). This solution was used for the following reactions.

(c) Synthesis of $NH_2$-$C_5$-DPPE

70 mg (100 μmol) of DPPE was suspended in 20 $m\ell$ of chloroform. The obtained suspension was further added with 50 $m\ell$ of TEA and 1 $m\ell$ (ca. 200 μmol) of Boc-5-aminovaleric acid succinimide ester, and stirred at 20°C overnight to replace succinimide residual group with DPPE. After reaction, the obtained solution was added with methanol and 3% citric acid solution to extract TEA, and dehydrated with anhydrous sodium sulfate. A solvent in the solution was removed with a rotary evaporator. Subsequently, 1.5 $m\ell$ of 1M hydrochloric acid/acetic acid was added to the obtained product for dissolution, and the resultant solution was allowed to stand at 37°C for one hour to remove the Boc group. The obtained solution was concentrated with a rotary evaporator, and repeatedly washed with methanol and chloroform to remove hydrochloric acid and sulfuric acid. Next, the obtained product was purified by a thin-layer chromatography (#5717, manufactured by Merck Co.) for separation utilizing a mixture solvent (chloroform/methanol = 7/3) as a developing solvent and silica gel as an absorbent. Yield of $NH_2$-$C_5$-DPPE was ca. 60%.

[2] Synthesis of bromoacetyl (BrAc)-NH-$C_5$-DPPE

140 mg (1 mmol) of bromoacetic acid was dissolved in 30 $m\ell$ of chloroform. The obtained solution was further added with 140 mg (1.2 mmol) of HSI and 250 mg (1.2 mmol) of DCCD, and reacted at room temperature for three hours, followed by removing a solvent therein by means of a rotary evaporator. The obtained product was added with 30 $m\ell$ of ethyl acetate, followed by filtering out the resultant white precipitant. The solvent contained in the product was removed again, and the product was re-dissolved in 10 $m\ell$ of chloroform.

Next, 1 $m\ell$ of the solution as obtained immediately above and 50 μ$\ell$ of TEA were added to ca. 10 $m\ell$ (50 μmol) of chloroform solution of $NH_2$-$C_5$-DPPE prepared in [1], and the resultant mixture was reacted at room temperature overnight. After the reaction, the solution was concentrated and subjected to a thin-layer chromatography for separation utilizing a mixture solvent (chloroform/methanol = 7/3) as a developing solvent to purify the product. Yield of BrAc-NH-$C_5$-DPPE was ca. 50%. This final product was diluted with chloroform to prepare a concentration of 1 mM.

[3] Preparation of Liposome

All lipids used was dissolved in a mixture solvent of chloroform and methanol (2/1).

Two hundred μ$\ell$ of 5 mM DPPC, 100 μ$\ell$ of 10 mM cholesterol, 50 μ$\ell$ of 1 mM BrAc-NH-$C_5$-DPPE prepared in [2], and 25 μ$\ell$ of 5 mM of stearylamine were introduced in a 10 $m\ell$ pear-shaped flask, and 2 $m\ell$ of chloroform was further added to the obtained mixture and sufficiently mixed. The solvent was removed from the resultant mixture with a rotary evaporator in a water bath at ca. 40°C. Two $m\ell$ of chloroform was further added in this flask, and sufficiently stirred, followed by removing the solvent with a rotary evaporator. This operation was repeated several times, and a thin layer of lipid was consequently formed on the inner surface of the flask. The flask was then transferred to a desiccator, and the solvent was completely removed with a vacuum pump for one hour.

Next, 100 $\mu\ell$ of 0.2 M carboxyfluoroscein (manufactured by Eastman Kodac Co., pH : 7.4, abbreviated CF hereinafter) was added in the flask. The atmosphere in this flask was replaced with $N_2$, and the flask was sealed and immersed in a water bath at ca. 60°C for ca. one minute. The flask was then violently stirred by a Vortex mixer until the lipid thin layer on the flask surface was completely removed. A multilamellar liposome suspension was prepared by this operation. A small amount of 0.01 M HEPES buffer (containing 0.85% $NaC\ell$, pH : 7.45, abbreviated HBS hereinafter) was added to this suspension, and the total amount of the obtained suspension was transferred to a centrifuge tube. This suspension was then centrifuged at 4°C at 15000 rpm for 20 minutes several times. Finally, the obtained liposome suspension was introduced to a serum tube (manufactured by Corning Co.) using 10 mM borate buffer (containing 0.85% $NaC\ell$, pH : 9.0, abbreviated BBS hereinafter), and centrifuged once, followed by removing a supernatant solution. The resultant liposome was preserved in a refrigerator until use for immobilizing monoclonal anti-DNA-RNA hybrid recognizing antibody (Fab').

[4] Modification of monoclonal anti-DNA-RNA hybrid recognizing antibody

One hundred $\mu\ell$ of solution of monoclonal anti-DNA-RNA hybrid recognizing antibody (subclass $IgG_1$) prepared from a mouse according to the method of W.D. Stuart et al. (Proc. Natl. Acad. Sci. USA., Vol. 78, No. 6, pp. 3571-3574, June 1981) was dialyzed with 0.1 M acetic acid buffer (pH 4.5), added with 10 $\mu g$ of pepsin (manufactured by Sigma Co.), and reacted at 37°C for one hour. SH group was introduced into the antibody by this reaction. Next, the obtained solution was subjected to HPLC to separate $F(ab')_2$ fragment. Ten mg of mercaptoethylamine hydrochloride was added to a 0.1 M phosphoric acid buffer (pH 6.0) containing $F(ab')_2$, and the mixture was reacted at 37°C for 90 minutes to separate only a protein fraction (Fab') containing free SH group by gel filtration (Sephadex G-25 gel, BBS solution). The volume of this protein fraction was 0.5 $m\ell$, and its OD 280 nm was 1.

[5] Immobilization of the monoclonal anti-DNA-RNA hybrid recognizing antibody to the liposome

The liposome suspension and the Fab' solution were mixed and stirred at 20°C for 44 hours for reaction. After that, the obtained mixture was washed with a gelatin-Veronal buffer solution (abbreviated GVB⁻ hereinafter) three times. The resultant liposome agent for gene detection was suspended in 2 $m\ell$ GVB⁻ and preserved at 4°C.

(B) Evaluation of the Liposome Agent for Gene Detection

Before the liposome agent as prepared above is utilized for gene detection, immune analysis was performed as follows in order to confirm that the antibody was immobilized on the liposome surface.

Rabbit anti-mouse IgG antibody (manufactured by Dako Co.) was diluted with $GVB^{2+}$ (which was formed by adding 0.5 mM $MgC\ell_2$ and 0.15 mM $CaC\ell_2$ in GVB⁻) to concentrations of 10-10⁵. Ten $\mu\ell$ of 10-fold diluted suspension containing the liposome agent and 50 $\mu\ell$ of 80-fold diluted solution containing complement (serum of guinea pig, complement value = 250) were added to 10 $\mu\ell$ of each diluted antibody solution, and the obtained mixture was reacted at 37°C for thirty minutes. The reaction was then terminated by adding 100 $\mu\ell$ of 0.01 M EDTA-Veronal buffer. With regard to each solution containing the rabbit anti-mouse IgG antibody of each concentration, the amount of CF flowed out was measured by a fluorescence spectrophotometer under the conditions that the excitation wavelength was 490 nm and the fluorescence wavelength was 520 nm.

Next, relative fluorescence intensity, i.e., lysis was calculated according to the following formula :

$$\text{relative fluorescence intensity} = \frac{Fe - Fo}{Fa - Fo} \times 100$$

where, Fe : fluorescence intensity measured, Fo : fluorescence intensity when $GVB^{2+}$ containing no rabbit anti-mouse IgG antibody was added (when no liposome was broken), Fa : fluorescence intensity when deionized water was added to break all of the liposomes. As standard values, fluorescence intensity of 10⁻⁷ and 10⁻⁸ CF solutions were used.

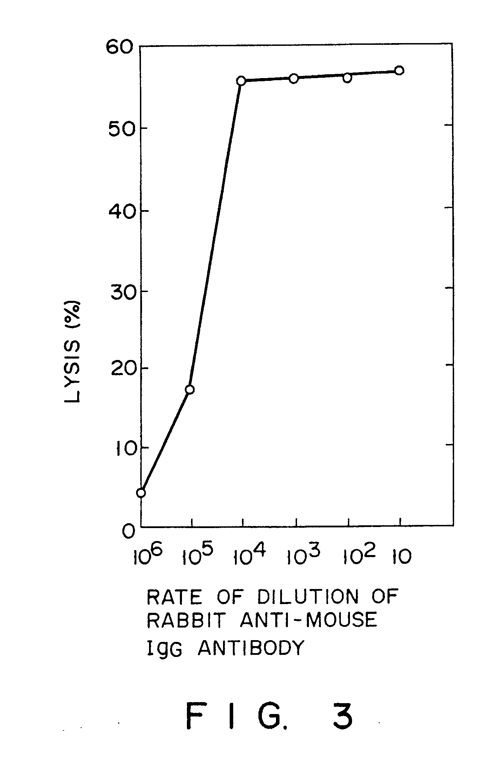

The results were shown in Fig. 3. As apparent from Fig. 3, the liposome agent was found to specifically react with the rabbit anti-mouse IgG antibody, and it was confirmed that the monoclonal antibody was immobilized on the liposome surface.

(C) Detection of HBV Gene in Human Serum

Detection of HBV gene human serum was performed by utilizing said liposome agent.

According to a general method, double-stranded DNA was extracted from human serum, cleaved with a restriction enzyme (Pst1) for fragmentation, and denatured to prepare single-stranded DNA. The single-stranded DNA and RNA gene probe against the HBV gene (manufactured by Daiichi Kagaku Co.) were reacted in a 1 m$\ell$ of GVB$^{2+}$(containing 50% formamide) at 60°C for thirty minutes for hybridization. The obtained solution was diluted 10-fold, added with a 100 $\mu\ell$ of the liposome agent suspension which had been diluted 10-fold with GVB$^{2+}$, and incubated at 37°C for thirty minutes. Next, 100 $\mu\ell$ of rabbit anti-nucleic acid antibody (which was formed by diluting rabbit serum 10-fold with GVB$^{2+}$) and 100 $\mu\ell$ of complement (which was formed by diluting serum of guinea pig 20-fold with GVB$^{2+}$) were added to the resultant solution, and the resultant mixture was incubated at 37°C for thirty minutes.

Subsequently, the amount of CF flowed out was measured by a fluorescence spectrophotometer under the conditions that the excitation wavelength was 490 nm and the fluorescence wavelength was 520 nm. Table 1 shows relative fluorescence intensity of 10 specimens. Here, #1-7 specimens are serum samples derived from hepatitis patients, and #8-10 were those from healthy people.

Table 1

| Specimen Number (#) | 1 | 2 | 3 | 4 | 5 | 6 | 7 | 8 | 9 | 10 |
|---|---|---|---|---|---|---|---|---|---|---|
| Relative Fluorescence Intensity (%) | 35 | 32 | 30 | 28 | 42 | 33 | 27 | 8 | 6 | 8 |

As apparent from Table 1, it was found that HBV gene was detected from the serum sample derived from the hepatitis patients only.

Moreover, sensitivity for detection by the liposome agent for gene detection was measured against a standard solution containing HBV gene. It was found that the HBV gene could be detected from ca. 1 pg of DNA, showing sensitivity as high as that obtained by utilizing gene probes labeled with radioisotopes.

Example 2

Liposome agent for gene detection was prepared by utilizing the same agents as those for Example 1 with the exception that a monoclonal antibody which specifically recognizes RNA (which was prepared from a mouse according to the W.D. Stuart method in the same manner as in Example 1) was used as an antibody immobilized on the liposome surface.

The obtained liposome agent was utilized to detect HBV gene in the same manner as in Example 1.

Table 2 shows relative fluorescence intensity of ten specimens (which were the same as in Example 1) as follows :

Table 2

| Specimen Numbers (#) | 1 | 2 | 3 | 4 | 5 | 6 | 7 | 8 | 9 | 10 |
|---|---|---|---|---|---|---|---|---|---|---|
| Relative Fluorescence Intensity (%) | 22 | 23 | 25 | 20 | 21 | 22 | 24 | 3 | 5 | 3 |

As apparent from Table 2, the result was almost the same as that obtained in Example 1. Moreover, sensitivity of the liposome agent for gene detection was measured against standard HBV gene. It was found that the standard HBV gene could be detected from ca. 1 pg of DNA in the same manner as in Example 1, showing a sensitivity as almost high as that obtained by utilizing gene probes labeled with radioisotopes.

Example 3

RNase H derived from E. coli was utilized as a substance immobilized on the surface of the liposome in this Example. RNase H was introduced site-directed mutagenesis at the active site by the method of K. Katayanagi et al. (Nature, Vol. 34, pp. 306-309, 20, Sep. 1990), thereby deactivating the activity as a nuclease. This RNase H was reacted with SPDP (manufactured by Pharmacia Co.) as a heterobifunctional cross-linking reagent and dithiothreitol as a reductant. As a result, SH group was introduced in RNase H.

The obtained RNase H having SH group was reacted with liposome, and thus the RNase H was immobilized on the surface of the liposome.

The obtained liposome agent was utilized to detect HBV gene in the same manner as in Example 1. Table 3 shows relative fluorescence intensity of ten specimens (which were the same as in Example 1) as follows. As apparent from Table 3, the resultant was almost the same as that obtained in Example 1.

## Table 3

| Specimen Numbers (#) | 1 | 2 | 3 | 4 | 5 | 6 | 7 | 8 | 9 | 10 |
|---|---|---|---|---|---|---|---|---|---|---|
| Relative Fluorescence Intensity (%) | 28 | 30 | 31 | 25 | 32 | 27 | 25 | 5 | 4 | 5 |

Additional advantages and modifications will readily occur to those skilled in the art. Therefore, the invention in its broader aspects is not limited to the specific details, and illustrated examples shown and described. Accordingly, various modifications may be made without departing from the spirit or scope of the general inventive concept as defined by the appended claims and their equivalents.

## Claims

1. Gene diagnosis comprising steps of :
   preparing single-stranded gene from a sample ;
   making said single-stranded gene to react with RNA or DNA gene probe which specifically reacts with the objective DNA or RNA, thereby forming DNA-RNA hybrid ;
   making said DNA-RNA hybrid to react with liposome agent for gene detection, where said liposome agent comprises a liposome which is formed of a phospholipid and/or a glycolipid, and contains a marker material therein, and a substance immobilized on the surface of said liposome which specifically recognizes DNA-RNA hybrid, DNA, or RNA ; and
   making the obtained composite of said DNA-RNA hybrid and said liposome agent to react with anti-nucleic acid antibody and complement, thereby breaking said liposome agent, followed by detection of the marker material flowed out.

2. Gene diagnosis comprising steps of :
   preparing single-stranded gene from a sample ;
   making RNA or DNA gene probe which specifically reacts with the objective DNA or RNA to react with said single-stranded DNA, thereby forming DNA-RNA hybrid ;
   making said DNA-RNA hybrid to react with liposome agent for gene detection, where said liposome agent comprises a liposome which is formed of a phospholipid and/or a glycolipid and contains a marker material therein, and a substance immobilized on the surface of said liposome which specifically recognizes the DNA-RNA hybrid ; and
   making a composite of said DNA-RNA hybrid and said liposome agent to react with anti-nucleic acid antibody and complement, thereby breaking said liposome agent, followed by detection of the marker material flowed out.

3. Gene diagnosis according to claim 1, characterized in that said RNA or DNA gene probe comprises a base sequence which is complementary to a specific region of the objective DNA or RNA.

4. Gene diagnosis according to claim 1, characterized in that said liposome includes cholesterol whose amount is 10-500 molar percent to the phospholipid and/or the glycolipid.

5. Gene diagnosis according to claim 1, characterized in that said marker material contained in the liposome is a fluorescent material, a luminescent material, a light-absorbable material, a glycoside and their oxidation enzyme, an ionic compound, a co-enzyme, or a radical compound.

6. Gene diagnosis according to claim 5, characterized in that said marker material contained in the liposome is a fluorescent material.

7. Gene diagnosis according to claim 6, characterized in that said fluorescent material is carboxyfluorescein.

8. Gene diagnosis according to claim 1, characterized in that said substance which specifically recognizes the DNA-RNA hybrid, DNA or RNA is an antibody or a part of an antibody.

9. Gene diagnosis according to claim 8, characterized in that said antibody is a monoclonal antibody.

10. Gene diagnosis according to claim 1, characterized in that said substance which specifically recognizes the DNA-RNA hybrid, DNA, or RNA is RNase or DNase, or a part of them.

11. Gene diagnosis according to claim 1, characterized in that said substance which specifically recognizes the DNA-RNA hybrid, DNA. or RNA is immobilized on the liposome surface with $-CO(CH_2)_mNHCOCH_2S-$ linkage, where $\underline{m}$ represents integer of 0-12.

12. Gene diagnosis according to claim 1, characterized in that said anti-nucleic acid antibody is obtained from rabbit serum.

13. Gene diagnosis according to claim 1, characterized in that said complement is obtained from guinea pig serum.

14. Liposome agent for gene detection comprising a liposome which is formed of a phospholipid and/or a glycolipid and which contains a marker material therein, and a substance immobilized on the surface of said liposome which specifically recognizes DNA-RNA hybrid, DNA or RNA.

FIG. 1

20

19

C P U

12

11

P

18

13:SINGLE-
STRANDED
DNA

P 18

P 18

P 18

18 P

17:COMPLEMENT

14:PILUENT

15:LIPOSOME
AGENT

16:ANTI-NUCLEIC ACID
ANTIBODY

F I G. 2

RATE OF DILUTION OF
RABBIT ANTI-MOUSE
IgG ANTIBODY

F I G. 3

European Patent
Office

**EUROPEAN SEARCH REPORT**

Application Number

EP 90 31 4249

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 336 454 (MILES INC.) <br> * Page 3, line 24 - page 4, line 25; page 8, lines 51-55 * | 1,8,9 | C 12 Q 1/68 <br> G 01 N 33/532 <br> G 01 N 33/58 |
| Y | | 3-7,10-14 | |
| Y | EP-A-0 312 212 (K.K. TOSHIBA) <br> * The whole document, especially page 9, lines 49-56; page 7, lines 31-40; claims * | 3-7,10-14 | |
| A | EP-A-0 135 159 (CETUS CORP.) <br> * Page 1, lines 24-31; page 2, line 26 - page 4, line 19; page 6, line 7 - page 9, line 4 * | 1 | |
| A | US-A-4 704 355 (D. BERNSTEIN) <br> * Abstract; column 5, lines 29-40; claims 1,7,14,15 * | 1 | |

TECHNICAL FIELDS
SEARCHED (Int. Cl.5)

C 12 Q
G 01 N

The present search report has been drawn up for all claims

| Place of search | Date of completion of the search | Examiner |
|---|---|---|
| THE HAGUE | 15-04-1991 | LUZZATTO E.R.P.G.A. |